## Europäisches Patentamt

## European Patent Office

## Office européen des brevets

(11) Publication number: **0 158 717**
**B1**

## EUROPEAN PATENT SPECIFICATION

(45) Date of publication of patent specification: **02.11.88**

(51) Int. Cl.⁴: **A 61 F 5/46**

(21) Application number: **84114540.2**

(22) Date of filing: **24.12.80**

(60) Publication number of the earlier application in accordance with Art. 76 EPC: **0 042 871**

(54) **A custom-made valved cervical cap.**

(30) Priority: **31.12.79 US 108319**
**26.06.80 US 163328**

(43) Date of publication of application:
**23.10.85 Bulletin 85/43**

(45) Publication of the grant of the patent:
**02.11.88 Bulletin 88/44**

(84) Designated Contracting States:
**DE FR GB**

(56) References cited:
**FR-A- 945 054**
**FR-A-2 282 853**
**US-A-2 551 618**
**US-A-3 307 222**
**US-A-3 404 056**
**US-A-4 007 249**

(73) Proprietor: **UNIVERSITY PATENTS, INC.**
**537 Newtown Avenue P.O. Box 6080**
**Norwalk, CT 06852 (US)**

(73) Proprietor: **CONTRACAP, INC.**
**999 Plaza Drive**
**Schaumburg, Illinois 60195 (US)**

(72) Inventor: **Goepp, Robert A.**
**5928 North Kilbourn**
**Chicago, IL 60646 (US)**
Inventor: **Freese, Uwe E.**
**238 North Forest**
**Oak Park, IL 60302 (US)**
Inventor: **Loeb, Marvin P.**
**7350 North Washtenaw Avenue**
**Chicago, IL 60645 (US)**

(74) Representative: **Heath, Derek James et al**
**Bromhead & Co. 30 Cursitor Street Chancery Lane**
**London EC4A 1LT (GB)**

Courier Press, Leamington Spa, England.

## Description

This invention relates to a valved cervical cap as defined in the preamble of claim 1.

Caps for cervix uteri as a birth control means have been known for a long time and have been found among the artifacts of antiquity. It has been reported that Aetius of Amida suggested for this purpose the use of a pomegranate shell cut into a hollow cup, and that beeswax discs fashioned to fit over the cervix have been used in Europe.

Modern cervical caps comprise a pre-formed rubber cap that is positioned over the cervix uteri to act as a sperm barrier. Such caps are manufactured in several sizes to accommodate the various uterine sizes normally encountered; however, with such caps the fit for a particular individual is inexact and necessarily a compromise. As a result, these caps often become dislodged during coitus and have to be removed periodically to accommodate normal uterine discharges. Thus, such caps are inconvenient to use and have not achieved a high degree of reliability.

Premanufactured cervical caps with valves are also known and are shown in U.S. Patent No. 2,836,177 to Sells, U.S. Patent No. 3,952,737 to Lippert et al. and German Patent No. 475,496 to Leopold. These caps also suffer from the aforementioned lack of stability and are subject to dislodgement during use.

U.S. Patent No. 4,007,249 to Erb describes a technique for custom fabrication of a cervical cap having a valve that is inserted during manufacture. The manufacturing expedients disclosed in this particular patent contemplate the painting of a liquid, polymerizable elastomeric material onto the cervix uteri followed by the polymerization of the painted material or, in the alternative, the use of a mold which retains a liquid, polymerizable elastomeric material in contact with the exocervical surface until it is polymerized.

The former expedient is impractical because the surface to which the liquid, polymerizable material is applied is wet with mucus and quite slippery, thus the applied material would fall off the exocervical surface due to gravity before polymerization of the material could take place and, in any event, it would be impossible to control the thickness of the applied polymerizable material during in situ polymerization. A cervical cap having a relatively non-uniform thickness is undesirable, however, because it is unstable and is likely to be dislodged in use. The second expedient disclosed in the Erb patent would also produce caps having an undesirable variable cap thickness that is likely to bring about dislodgement.

A further shortcoming of the caps shown in the Erb patent is that the prefabricated valving means utilized are of the leaflet or flap type. In such valves a viscous droplet of cervical mucus could hold the valve in an open position for an undesirably long time period, thereby providing an access aperture for sperm and defeating the very purpose of the cap. Moreover, inasmuch as the polymerizable material of the cap is in a fluid state when it surrounds the prefabricated valve, some of the fluid material may become enmeshed with the valve and interfere with its intended valving action after the material has polymerized.

U.S. Patent No. 4,007,249 to Erb also mentions a technique disclosed initially by F. A. Wilde in 1838 in Das Weibliche Gebar-Unvermogen according to which a cervical cap allegedly can be made from a special wax impression of the vaginal portion of the cervix. As recognized by Erb, such a technique cannot produce an identical, negative-image, cervix-conforming inside surface because the cervix would be deformed while the wax impression is being made. The uterus is suspended in the lower abdomen by ligaments, is easily movable, and would tend to move up into the abdomen even with a gentle force applied to the cervix. As a result, accurate registration would be prevented by such a movement with attendant lack of stability for the cervical cap produced in such manner.

The techniques described by Erb are also likely to suffer from the same drawback, albeit for a different reason. In particular, in practicing these techniques the vaginal wall has to be expanded using a vaginal speculum or similar implement in order to expose the cervix during cap molding. This expedient tends to distort the cervix as well, elongating it along an imaginary line between the tips of the inserted, open vaginal speculum blades and shortening the cervix along an imaginary line at right angles to the imaginary line between the tips of the speculum blades. The attendant cervical distortion exceeds the limits for prosthetic stability of the cervical cap that is produced.

Accordingly, while a stable, well-fitting cervical cap can be an effective birth-control device, heretofore it has not been possible to produce a cervical cap that has the requisite stability against dislodgement and that can be worn for extended periods of time such as months, or even years, without removal.

With these considerations in mind, the present invention is directed to a valved cervical cap comprising a cup-shaped elastomeric shell having a convex outer surface and a concave inner surface that is substantially complementary with contiguous surface of portio vaginalis cervicis in its actual configuration when in contact therewith, the shell defining an aperture at the apex thereof, characterised in that the shell has a depth sufficient to receive a major portion of said portio but with the periphery of said shell terminating short of fornices vaginae, and an elastomeric web over the aperture and secured to the shell's outer surface about the aperture defines a passageway communicating with the aperture and providing a discharge port along the convex outer surface and offset from the aperture, the web conforming to and co-acting with a contiguous portion of the convex outer surface to provide a one-way valve for uterine discharge entering the aperture from within the shell.

Preferably a skirt portion is integral with the shell, depending from the shell at the periphery of the shell, and having a thickness that is less than the thickness of the shell, the skirt portion having a plurality of spaced slits extending from the peripheral edge of the skirt portion.

The invention also extends to a method of making a valved cervical cap, characterized by the steps of: (a) applying a physiologically-tolerable, hardenable paste to cervix uteri in normal configuration and forming an impression of the cervix; (b) replicating the cervix as a rigid mould; (c) postioning a blank for a valved cervical cap made of a thermoplastic elastomeric material over the rigid mould; (d) applying heat to the blank in an amount sufficient to soften the material; (e) pressing the softened material against the rigid mould; (f) cooling the pressed, softened material; and providing the resultant cap-shaped product with an aperture at its apex and with an elatomeric web over the aperture.

Preferably, in the method according to the present invention, an impression tray is used which is suitable formaking an impression of a cervix uteri and which comprises (a) a hollow cup for receiving the cervix and having a flexible wall terminating in a peripheral, outwardly flared anterior lip and a bottom aperture; (b) a posterior sleeve integral with said cup and surrounding said bottom aperture; and (c) a hollow elongated stem having a proximal end thereof received within said posterior sleeve and defining a passageway communicating with the interior of said cup.

In one desirable example of a method according to the present invention, an implement is used for making an impression of a cervix uteri which comprises, in combination, an impression tray and a syringe having an elongated nozzle received within said tray, said tray comprising a hollow cup for receiving the cervix and having a flexible wall terminating in a peripheral, outwardly flared anterior lip and a bottom aperture; a posterior sleeve integral with said cup and surrounding said bottom aperture; and a hollow elongated stem having a proximal end thereof received within said posterior sleeve and defining a passageway communicating with the interior of said cup; and said syringe comprising a hollow body portion, a movable plunger within said body portion, and an elongated nozzle communicating with said body portion and adapted to be received within said passageway of the hollow elongated stem.

To make an impression of the cervix, the impression tray is positioned over the cervix with the stem protruding from the vagina. A physiologically tolerable, hardenable paste is dispensed from the nozzle of the syringe when inserted into the stem. The paste is distributed over the cervix and within the confines of the hollow cup of the tray. The dispensed paste is permitted to harden in situ, and thereafter the tray, containing the fixed cervical impression, is removed from the vagina. A replica of the cervix is then cast from the obtained impression and hardened. Thereafter the cervical cap is produced by positioning the aforementioned blank over the replica, softening the blank by application of heat thereto, and then pressing the heat-softened blank against the replica by vacuum, air pressure, or otherwise. After the surface of the softened blank contiguous with the replica has been deformed to assume a contour substantially complementary to that of the replica, the deformed blank is cooled, removed from the replica, and trimmed to a desired size.

The valved cervical cap embodying the present invention is a non-invasive and entirely passive birth control device. The cap does not interfere with the normal hormonal balance of the individual wearer and thus avoids the side effects commonly encountered with hormonal contraceptives.

Inasmuch as the cap is not placed within a human body like an intrauterine device but, instead, is positioned on the exocervical surface of cervix uteri, it does not have to be sterilized. Likewise, no foreign body reaction and attendant rejection has been encountered.

The cap may be worn for extended periods of time, no preplanning, anticipation, or periodic attention is needed. Thus, there is no interference with the spontaneity of sexual activity. Additionally, the presence of the cap may protect the cervix, uterus and Fallopian tubes from undesirable external influences and infections.

The cap embodying this invention can be fabricated in a relatively short time period, of the order of about five minutes, using a replicated cast of the prospective user's cervix uteri. Little technical skill is required for fabrication.

The one-way valve included in the present cap provides the further advantages of reliability and increased sperm travel distance in the unlikely event that sperm does enter the space between the web and the shell, since the possible sperm entry region is distant from the shell aperture. The normal cyclical changes in the viscosity of cervical mucus do not affect the efficacy or the barrier characteristics of the valve. In contradistinction to the previously known leaflet or flap-type valves which could be held open by a viscous droplet of cervical mucus, the present valve design causes droplet-like cervical mucus descending from the cervical os to flatten out into a very thin film, which does not hold open the valve itself.

European Application No. 81900386.4 (publication Nos. EP-A-0042871 and WO-A-81/01791) contains subject matter which relates to the claims of this application.

Brief Description of the Drawings

In the drawings,

FIGURE 1 is an elevational view, partly in section, of a portion of the internal organs of the female reproductive system with a cervical cap embodying the present invention in place;

FIGURE 2 is an enlarged perspective view of a valved cervical cap embodying the present invention;

FIGURE 3 is a plan view of a blank suitable for fabricating a cervical cap embodying this invention;

FIGURE 4A is a sectional view taken along plane 4-4 in Figure 3;

FIGURE 4B is a sectional view similar to that in Figure 4A but showing an alternate cross-sectional configuration for the valve means in a cervical cap of this invention.

FIGURES 5 and 6 illustrate devices suitable for practicing the present invention;

FIGURE 7 is a side elevational view showing a cast replica of the cervix of female uterus;

FIGURE 8 is an enlarged sectional elevational view illustrating the manufacture of a cervical cap in accordance with the present invention;

FIGURE 9 is a plan view showing another type of blank suitable for fabricating a cervical cap with a deformable margin in accordance with the present invention;

FIGURE 10 is a sectional view taken along plane 10-10 in FIGURE 9;

FIGURE 11 is a bottom view of the blank shown in FIGURE 9;

FIGURE 12 is an enlarged perspective view of a valved cervical cap embodying the present invention and fabricated from the blank shown in FIGURES 9-11, inclusive.

FIGURE 13 is an elevational view, partly in section, of a portion of the internal organs of the female reproductive system with a cervical cap having a deformable margin in place;

FIGURE 14 is an enlarged perspective view of a valved cervical cap with a deformable margin;

FIGURE 15 is a plan view of another blank suitable for fabricating a cervical cap with a deformable margin;

FIGURE 16A is a sectional view taken along plane 16-16 in Figure 15; and

FIGURE 16B is a sectional view similar to that in Figure 16A but showing an alternate cross-sectional configuration for the valve means in a cervical cap of this invention.

Description of Preferred Embodiments

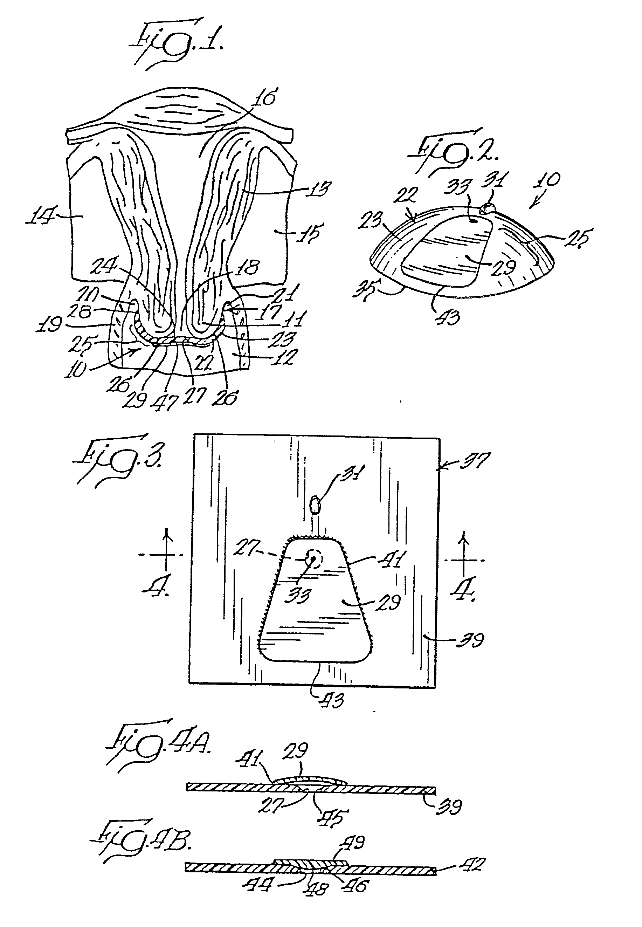

Referring to FIGURE 1, valved cervical cap 10 is shown positioned on exocervical surface 17 of cervix uteri 11, i.e., on the portio vaginalis cervicis or that portion of uterus 13 that protrudes into vagina 12. Uterus 13 is supported by broad ligaments 14 and 15, and defines fundus 16 that terminates in cervical os 18. Vaginal wall 19 and cervix 11 together define the fornices vaginae, i.e., lateral fornices 20 and 21 as well as the anterior and posterior fornices (not shown).

Cap 10 is a cup-shaped elastomeric shell 22 having convex outer surface 23 and concave inner surface 24. Surface 26 of the portio vaginalis cervicis is not distorted by cap 10 and is in mating contact with concave inner surface 24 at least along band 25 of shell 22 which is of substantially uniform rigidity. In the area of contact, concave inner surface 24 is substantially complementary with the contiguous surface 26 of the portio vaginalis cervicis. preferably the thickness of shell 22 is substantially uniform, at least along peripheral band 25, for optimum stability of cap 10 when in place. Also, it is preferred that the band of substantially uniform thickness constitutes a major portion of shell 22.

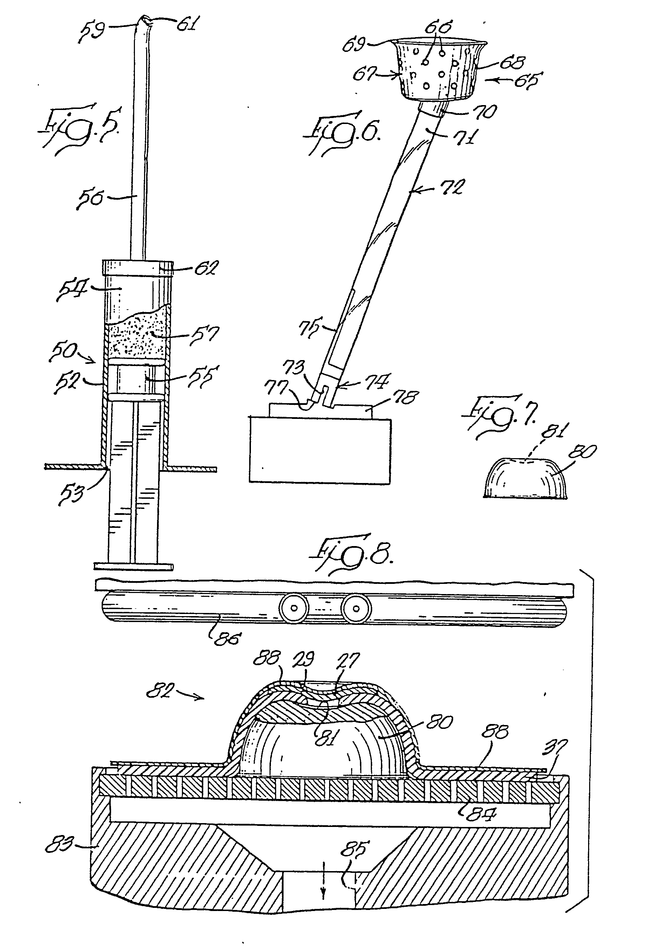

Aperture 27 is provided in elastomeric shell 22 at the apex thereof and, as shown in FIGURE 1, is positioned adjacent to cervical os 18 when cap 10 is in place. An external cover for aperture 27 is provided by elastomeric web 29 that is integral with shell 22. Web 29 is secured to shell 22 about a major portion of its periphery but also having an unsecured or free edge portion (FIGURE 2) that together with adjacent surface portion of convex surface 23 defines a discharge port or external opening 43. Web 29 lies against shell 22 and substantially conforms to the contour thereof. In some instances web 29 may also be under tension and/or biased against convex surface 23. Web 29 and the covered portion of outer surface 23 together define a pocket that communicates with aperture 27 and forms a one-way valve extending from aperture 27, between web 29 and surface 23, and defining discharge port 43 in a direction away from the apex of shell 22, and thus spaced from aperture 27. Preferably the defined discharge port is situated at least about one aperture diameter away from aperture 27, and more preferably, is situated near or at shell periphery 35.

This one-way valve opens under increased uterine pressure and permits the passage of fluids such as mucus, menstrual flow, and the like from fundus 16 to vagina 12 without disturbing the positioning of cervical cap 10 and without permitting the entry of sperm into fundus 16. Additionally, the precise fit of cervical cap 10 onto the portio permits mucus to flow around it; however, at the same time the forces generated by the flow of mucus assist in orienting and in holding cap 10 firmly in place.

As shown in FIGURE 2, optionally cap 10 can be provided with an integral tactile orientation marker 31 for the purpose of facilitating orientation of cap 27 upon insertion, as well as enabling the wearer to periodically assure herself that cap 10 remains properly positioned. Visual indicia 33 may also be provided on elastomeric web 29 in some instances for the purpose of facilitating the locating of aperture 27 underneath web 29 during manufacture of cap 10 as will be described in detail hereinbelow.

Shell 22 is dimensioned so as to have a depth sufficient to receive a major portion of the portio as illustrated in FIGURE 1; however, periphery 35 of shell 22 terminates short of the fornices vaginae so as to guard against dislodgment of cap 10 during activity that may cause uterus 13 to shift position and/or to deform or distend the normal configuration of the fornices. To avoid dislodgment of the cap during momentary, severe uterine displacement it is important that peripheral edge 35 is sufficiently elastic to remain in contact with the exocervical surface contiguous therewith even during such momentary uterine displacement. For this purpose it is preferable that peripheral edge 35 be feathered, i.e., tapered or beveled as shown in FIGURE 1 at 28.

Cap 10 preferably is positioned on cervix uteri 11 so that discharge port or external opening 43 of the valve defined by web 29 in conjunction with convex outer surface 23 is located in or near the posterior fornix and also at a location on surface 23 that is closer to shell periphery 35 than to the apex of the shell. In the embodiment illustrated in FIGURE 2 discharge port 43 is substantially coincidental with periphery 35 and preferably is situated no more than about 1 to about 3 millimeters from periphery 35. This results in a manifold increase in the distance that sperm must travel to reach cervical os 18.

A portion of the increased distance for sperm travel is in vagina 12 where the environment is very inhospitable to sperm mobility due to the relatively low ambient pH. Usually sperm can survive in the vaginal vault only for a time period of about one to two hours. Inasmuch as sperm can move at a velocity of about one to two millimeters per minute, the substantial increase in the distance that sperm must travel with cap 10 in place alone markedly reduces the likelihood of fertilization. Sperm travel to cervical os 18 via the valve means in cap 10 is possible only in close proximity to the walls defining the valve means. Inasmuch as at least shell 22, and preferably both shell 22 and web 29 are of a thermoplastic elastomeric material, which materials exhibit an inhibitory effect on sperm motility, the likelihood of sperm reaching cervix uteri 11 is further reduced.

Shell 22 can be made of a wide variety of thermoplastic elastomeric materials, the so-called thermoplastic elastomers, such as polyolefin blends, styrene/elastomer block copolymers, copolyesters, and polyurethane block copolymers. While these thermoplastic elastomers differ chemically, their morphology is similar. Blocks or domains of relatively hard thermoplastic constituents link elastomeric constituents in a network that behaves like a chemically crosslinked rubbery structure. At forming temperatures the relatively hard thermoplastic domains of the structure soften and allow the polymeric material to flow. Upon cooling, these relatively hard domains resolidify and re-establish the rubberlike, elastic structure.

Thermoplastic elastomers that exhibit a surface charge provide a further advantage for the present purposes in that the presence of such a charge on the fabricated shell and/or web tends to inhibit sperm motility.

For purposes of the present invention, the shell materials particularly suitable are the styrene/elastomer block copolymers such as those commercially available from the Shell Chemical Company, Oak Brook, Illinois, under the designation "Kraton" and "Kraton G" and described in U.S. Patent No. 3,231,635 to Holden et al. These styrenic thermoplastic elastomers are block copolymers of polystyrene and an elastomer such as polyisoprene, polybutadiene, ethylene-propylene, or ethylenebutylene rubber.

While thermoplastic elastomeric materials of varying hardness may be used to fabricate shell 22, for optimum stability against dislodgment preferably the material should be harder than the cervical tissue that comes in contact therewith, yet the hardness should not be so high as to cause discomfort to the wearer or her consort. preferably the shell material has a Shore A durometer hardness value of about 35 to about 70, and more preferably of about 45 to about 60.

The shell material can be opaque, semitransparent or transparent; however, for ease of handling or positioning during manufacture of the cervical cap a transparent or clear material is preferred.

Web 29 can be made from the same elastomeric material or from a different elastomeric material, as long as web 29 can be secured to shell 22. For ease of manufacture it is preferred to have web 29 of the same elastomeric material as the shell material but thinner. The shell-to-web thickness ratio usually is about 7:1 to about 3:1 and preferably about 5:1. In a typical cervical cap embodying the present invention the shell thickness is about 1.5 millimeters and the web thickness is about 0.3 to about 0.4 millimeters.

The relative thicknesses of the shell and the integral web in each instance depend on a variety of factors such as the manufacturing procedure, forming temperatures, modulus of elasticity, and the like considerations. In general, however, the web thickness is selected so as to provide a valve-opening pressure of about 10 to about 15 millimeters of mercury for the one-way valve formed by the coaction of web 29 with the outer surface 23 of shell 22 contiguous therewith.

The material for web 29 need not be thermoplastic as long as it exhibits the desired elasticity and can be secured to shell 22. Not only heat sealing or ultrasonic bonding but other bonding means, e.g., adhesive bonding, can be utilized as well. In addition to the aforementioned thermoplastic elastomers, the web portion of the present valved cervical cap can be made from materials such as natural rubber, silicone rubber, polyurethanes, fluorocarbon rubbers, styrene-butadiene rubbers, and the like.

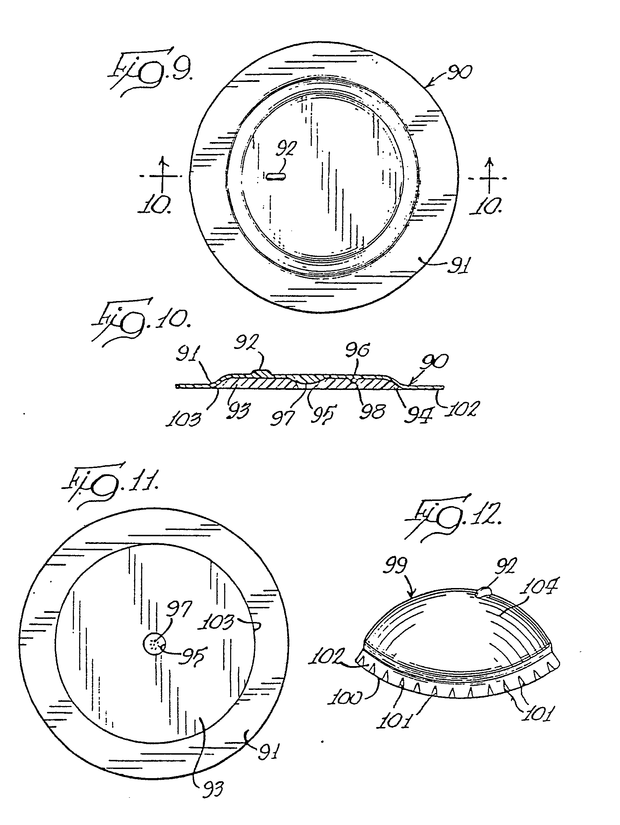

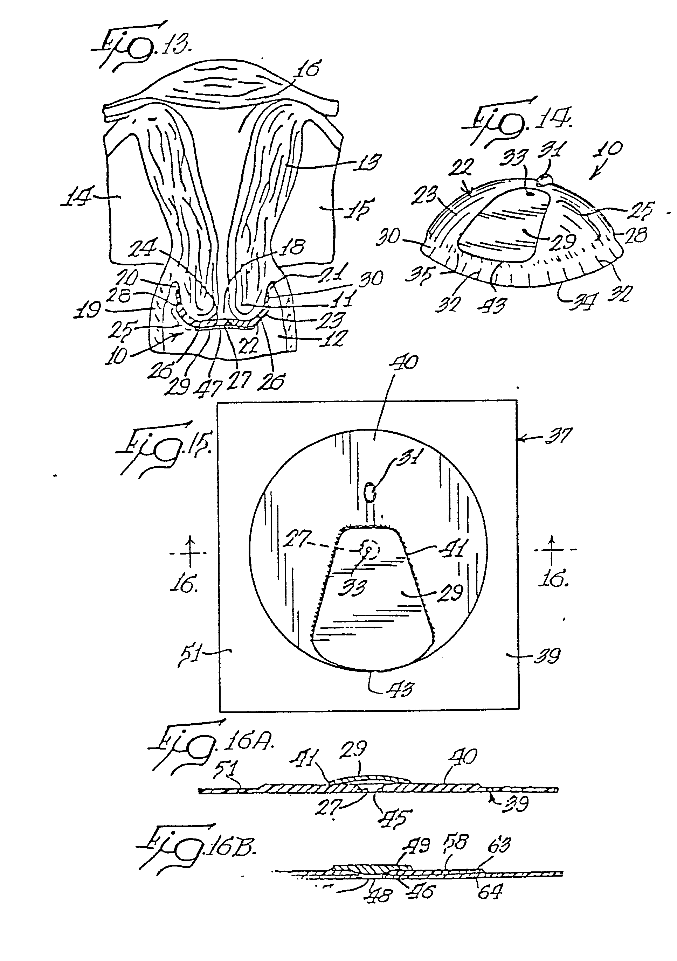

Valved cervical cap 10 can be fabricated by molding a blank of the type shown in FIGURES 3 and 4A wherein blank 37 comprises substantially planar sheet 39 of elastomeric material. Aperture 27 in the central region of sheet 39 is covered by elastomeric web 29. The contour of web 29 is not critical and can be generally fan-like, bell-shaped, trapezoidal, or circular, for example, as long as the web substantially conforms to the molded shell after the cap has been fabricated.

Web 29, in turn, is secured to sheet 39, e.g., by bonding peripheral region 41 thereof. The securement of web 29 to sheet 39, and thus ultimately to cap 10, is sufficient to permanently attach web 29 to sheet 39 but provides an unsealed, i.e., free, edge portion which serves to define, in part, external valve opening or discharge port 43. In essence, web 29, when secured to sheet 39, defines a pocket or pockets communicating with underlying aperture 27 and provides a one-way passageway or channel between web 29 and that portion of sheet 39 which ultimately becomes a web-covered portion of the convex outer surface 23 for cap 10, terminating in discharge port 43 that is situated at least one aperture diameter away from aperture 27. Preferably the cross-sectional area of the defined passageway increases with increasing distance from aperture 27 as measured along convex surface 23 of shell 22. Thus, in case of a trapezoidal web, for instance as shown in FIGURE 3, the relatively shorter of the two substantially parallel sides of the trapezoid is positioned nearer to aperture 27 than the relatively longer parallel side, and bonded peripheral region 41 extends about the major portion of the periphery of aperture 27. The diameter of aperture 27 is usually about 5 to about 10 millimeters.

Aperture 27 can be a through-aperture with rectangular edges, if desired. However, during fabrication, a relatively thin, thermoplastic, elastomeric web may be drawn or forced into aperture 27 too far and may be excessively deformed or even perforated at the line of contact with a rectangular, sharp edge defining aperture 27. The use of such relatively thin webs, if desired, is facilitated if the aperture-defining edge portion adjacent to web 29 is beveled as at 45 in FIGURE 4A or rounded as at 47 in FIGURE 1. In this manner the likelihood that web 29 may be perforated or unduly distorted during fabrication is reduced. Tactile orientation marker 31 is positioned adjacent to a sealed juncture of web 29 and sheet 39.

An alternate, suitable one-way valve configuration utilizing a relatively thin web is illustrated in FIGURE 4B. In this particular embodiment, aperture 44 in thermoplastic elastomeric sheet 42 is covered by web 49 having unitary protuberance 48 extending into aperture 44 and contacting beveled surface 46, i.e., the web 49 is thicker in the region of aperture 44. When a custom, valved cervical cap is fabricated using a cap blank of the type shown in FIGURE 4B, protuberance 48 will abut beveled surface 46 and thus will prevent excessive deformation of the web itself during fabrication.

It should be noted, however, that even in the case of a substantially uniformly thick web some deformation into the shell aperture, such as aperture 27 (FIGURES 1, 3 and 8), is not objectionable as long as web integrity is not impaired. Such deformation, as best seen in FIGURE 8, also can provide a unitary protuberance that projects into the aperture and enhances the valving action. In addition, the web may be relatively thicker in the region overlying the aperture.

In some instances, particularly when sheet 39 is relatively thick, e.g., of the order of about 2.5 millimeters or more, it is desirable to extend bevel 45 on the aperture edge portion substantially to the juncture 41 of sheet 39 and web 29 so as to provide a substantially uniform thickness throughout. Alternatively, or in addition, the region surrounding aperture 27 and covered by web 29, in the region between juncture 41 and aperture 27 may be countersunk or thinned out for the same purpose.

A prefabricated blank of the type described offers the substantial advantages of ease of custom cap fabrication by a practitioner associated with the practicing physician or with a medical laboratory.

For optimum fit, concave inner surface 24 of cap 22 is substantially complementary with the surface of the portio vaginalis cervicis contiguous therewith. While it is desirable that concave inner surface 24 projects slightly toward cervical os 18 about the periphery of aperture 27, i.e., has local convexity in the aperture region complementary to the concavity of the cervix in the region surrounding cervical os 18, no portion of surface 24 enters cervical os 18. To this end, the cervix in its normal, undistorted configuration is first replicated as a rigid mold, e.g., in plaster, and the obtained replica is then utilized to mold the cervical cap.

Initially an impression of cervix uteri is made using a physiologically tolerable, hardenable paste. Suitable for this purpose is an aqueous paste made from dental impression powder, e.g., using an algin derivative, usually an alginate such as sodium alginate.

The cervical impression can be made utilizing the implements shown in FIGURES 5 and 6. Syringe 50, shown in FIGURE 5, includes hollow body portion 52 having an open end 53 and opposed dispensing end 54 that terminates into elongated nozzle 56 in communication with hollow body portion 52. Plunger 55 is positioned within hollow body portion 52 and is adapted to dispense the syringe contents through nozzle 56. Impression paste 57, e.g., aqueous alginate paste, is contained within hollow body portion 52. To facilitate the distribution of impression paste 57 over the cervix during the course of making the impression, it is preferable to have a nozzle the distal end 59 of which is offset from the longitudinal axis of the nozzle itself. In this manner nozzle discharge port 61 is situated in a plane that is at an acute angle, preferably at an angle of about 45 degrees, with respect to the longitudinal axis of nozzle 56, and can dispense impression paste 57 laterally over the cervix in all directions by simply rotating syringe 50. In an alternate manner, nozzle 56 can be rotatably mounted on syringe body 52 by means of nozzle-bearing hub 62 that is rotatable with respect to syringe body 52.

Preparatory to making a cervical impression as contemplated by the present invention, vagina 12 is expanded using a vaginal speculum so as to expose the cervix. Thereafter impression tray 65 (FIGURE 6) is inserted through the speculum and into the vagina, and positioned so that hollow cup 67 thereof envelops the cervix. Cup 67 has a flexible, preferably transparent, wall 68 made from an elastomeric material such as silicone rubber, polyurethane, or the like, that terminates in a peripheral, outwardly flared, anterior lip 69 the purpose of which is to keep out adjacent vaginal wall. Wall 68 and lip 69 are also designed so as to trap the cervix and to prevent its upward rotation with attendant closure of the anterior fornix space when the patient is in a supine position while the cervical impression is made.

Flexible wall 68 is further provided with a plurality of spaced perforations such as perforation 66. Cup 67 at the bottom thereof is provided with a bottom aperture surrounded by posterior sleeve 70. Proximal end 71 of hollow, elongated stem 72 is received within sleeve 70. Stem 72 defines a passageway that communicates with the interior of cup 67. A pair of opposed mounting notches, such as notch 73, are provided in distal end 74 of stem 72. Also at or near distal end 74 marker means 75 is provided on stem 72 for indicating a preselected orientation of cup 67.

While cup 67 and stem 72 can be coaxial, for ease of manipulation and insertion during the making of a cervical impression, and also to facilitate the entrapment of the cervix, it is preferred that the longitudinal axes of cup 67 and stem 72 intersect at an included angle of about 15 to about 25 degrees, and more preferably about 20 degrees.

To make a cervical impression once tray 65 is appropriately positioned enveloping the cervix, nozzle 56 is inserted through hollow stem 72. Preferably, length of nozzle 66 is selected so as to extend for substantially the length of stem 72. More preferably distal end 59 projects into cup 67 through the bottom aperture thereof but remains spaced from the cervix. Hub 62 (FIGURE 5) on syringe, upon abutting open end 77 of stem 72, provides a positive stop that prevents nozzle aperture 61 from coming in contact with the cervix. In instances where nozzle 56 is longer than stem 72, appropriate stop means can be provided on nozzle 56 itself or within stem 72 at the proximal end 71 received within sleeve 71.

Impression paste 57 is then dispensed from syringe 50 and distributed so as to cover the cervix, and speculum is removed from the vagina without removing tray 65, and the paste permitted to set with the cervix in its natural undistorted shape. The length of stem 72 is selected long enough to permit the easy removal of the speculum without disturbing the position of tray 65. As the impression paste fills cup 67, minor amounts of the paste exude through perforations 66 and, upon setting, anchor the formed impression within tray 65.

After the paste has set, usually within about 90 seconds, tray 65 together with the cervical impression contained therewithin is lifted from the cervix and placed onto vibrating table 78 for casting a replica of the cervix.

The casting operation entails pouring a slurry of plaster of Paris, or like casting material, into tray 65 while the latter is vibrated to permit any trapped air to rise to the surface and to assure that each and every crevice of the formed impression is filled with the slurry. After the slurry has hardened, replica 80 (FIGURE 7) is removed from tray 65 and is ready for use in fabricating cervical cap 10.

To this end vacuum molding techniques, e.g., drape forming, can be readily utilized; however, any other molding technique can be utilized as long as the material to be molded can be made to conform closely to the cervical replica, for example, by means of positive air pressure. For purposes of illustration a preferred vacuum molding technique will be described in detail hereinbelow.

Referring to FIGURE 8, vacuum molding apparatus 82 comprises base 83 that supports foraminous plate 84 and heat source 86, e.g., a calrod heating element or infrared lamp, in a spaced relationship relative to plate 84. Alternatively, heat can be supplied by a stream of hot air. In the case of a radiant heat source, such as an infrared lamp, the heat-radiating surfaces can be contoured so as to provide a substantially uniform distance between the heat source and the blank portion nearest thereto. Channel 85 in base 83 communicates with a vacuum source (not shown) and permits vacuum to be drawn through plate 84.

Replica 80 is positioned on plate 84 and blank 37 positioned thereover so that visual indicia 33 is superposed over cervical os region 81 of replica 80. A release agent, e.g., an aerosol spray of lecithin commercially available under the designation "Pam" from Boyle-Midway, Inc., New York, N.Y. 10017, is applied to the exposed surface of blank 37, and between the web and the underlying sheet and the blank is then covered with thin protective sheet 88, e.g., a polyethylene terephthalate film, or the like. Preferably sheet 88 is perforated in order to facilitate the vacuum forming operation. Heat source 85 is then turned on and heat applied to sheet 37 until it begins to soften and conform to replica 80 by its own weight. Thereafter the vacuum source is turned on and vacuum drawn through foraminous plate 84 so as to press softened sheet firmly against replica 80. Heat source 86 is then turned off or removed, and the formed sheet is permitted to cool while vacuum is maintained. Once cooled to a permanent contour, sheet 37 is removed from replica 80, and the formed cervical cap excised therefrom and trimmed to the desired shell depth. The specific shell depth in each instance will depend on the contour of the portio vaginalis cervicis in a given case; however, the shell peripheral edge, i.e., periphery 35, should not extend into the fornices vaginae. The feathering or beveling of shell periphery 35 is usually done during the final trimming operation.

Valved cervical caps embodying the present invention, made from styrene/elastomer block copolymers, and having the general configuration shown in FIGURE 2 have been satisfactorily worn by sexually active female volunteers for a cumulative time period in excess of 290 months.

The cervical cap shown in Figures 13 and 14 is substantially the same as the cervical cap shown in Figures 1 and 2 except that it has a flexible skirt portion 30. For ease of understanding and for clarity, the parts and components of the cervical cap shown in Figures 13 and 14 have been given the same part numbers as similar parts and components of the cervical cap shown in Figures 1 and 2.

Flexible skirt portion 30 provides a deformable margin that depends from shell 22 and is substantially thinner than the thickness of peripheral band 25, i.e., no more than about one-half the thickness of band 25.

Skirt portion 30 can be unitary with shell 22 or can be made from a separate strip of the same or different elastomeric material secured to shell 22 at the peripheral edge thereof by heat sealing, adhesive bonding, ultrasonically, or in any other convenient manner. At the juncture of shell 22 and skirt portion 30, edge portion 28 of shell 22 is featured, i.e, beveled or tapered, so as to provide a relatively smooth, gradual transition from one material thickness to another.

Aperture 27 is provided in elastomeric shell 22 at the apex thereof and, as shown in FIGURE 13, is positioned adjacent to cervical os 18 when cap 10 is in place. An external cover for aperture 27 is provided by elastomeric web or web portion 29 that is integral with shell 22. Web 29 is secured to shell 22 about a major portion but not the entirety of its periphery so as to provide an unsecured or free edge portion (FIGURE 14) that together with adjacent surface portion of convex surface 23 defines a discharge port or external opening 43. As shown in FIGURE 14, web 29 covers only a portion of shell 22; however, web 29 can also cover the entire external surface of shell 22. If desired, web 29 may extend also beyond shell periphery 35 up to peripheral edge 34 of skirt portion 30.

As best seen in FIGURE 14, flexible skirt portion 30 is provided with a plurality of spaced slits 32 that begin at peripheral edge 34 of skirt portion 30 and which can extend toward the apex of shell 22 substantially at right angles to peripheral edge 34. Slits 32 may extend substantially across the entire width of skirt portion 30 or may terminate short of beveled portion 28 of shell 22. In one preferred embodiment of this invention the slits extend at least one-half the width of the skirt portion. The spacing between adjacent slits depends on the flexibility of the skirt portion and may vary in the range of about 2 millimeters to about 10 millimeters. More preferably slits 32 are spaced about 4 to about 6 millimeters apart and are at least about 1.5 millimeters long.

For optimum retention of the cap, it is desirable to maximize the contact area between the cap and the exocervical surface. To this end, skirt portion 30 integral with shell 22 performs a very useful function in that further contact area is provided. Additionally, the relatively thin cross-section of flexible skirt portion 30, and slits 32, permit a localized deformation of skirt portion 30 upon uterine displacement or distortion without a material reduction in the contact area available for cap retention. Skirt portion 30 is preferably made out of the same material as shell 22, but may be made out of different materials or have different physical properties.

Valved cervical cap 10 embodying the present invention can be fabricated by molding a blank of the type shown in FIGURES 16A and 16B wherein blank 37 comprises substantially planar sheet 39 of elastomeric material. Sheet 39, in turn, comprises a central embossed region or land 40 of generally circular configuration from which shell 22 is formed surrounded by a relatively thinner region 51 from which skirt portion 30 is ultimately formed. Typically the central embossed region is about 1 to about 2 millimeters thick and the planar surrounding region is about 0.2 to about 0.4 millimeters thick, i.e., the surrounding, relatively thinner region has a thickness that is no more than about one-half of the thickness of the embossed region. Preferably the thickness ratio of the embossed region to the planar surrounding region is about 3:1 to about 7:1. Typically, the embossed region or land 40 has a diameter of about 25 millimeters to about 50 millimeters. Aperture 27 in the central region of sheet 39 is covered by elastomeric web 29. The thickness ratio of the embossed region to the overlying web preferably is about 7:1 to about 3:1, and more preferably is about 5:1. The contour of web 29 is not critical and can be generally fan-like, bell-shaped, trapezoidal, or circular, for example, as long as the web substantially conforms to the molded shell after the cap has been fabricated and defines a channel terminating at the distal end thereof in a discharge port that can accommodate the uterine discharges.

For a bell or fan-shaped web 29, for instance as shown in FIGURE 15, the bonded peripheral region 41 extends about the major portion of the periphery of aperture 27 and the maximum cross-sectional area for the defined passageway is at discharge port 43. The diameter of aperture 27 is usually about 5 to about 10 millimeters.

An alternate, suitable one-way valve configuration utilizing a relatively thin web is illustrated in FIGURE 4B. In this particular embodiment, aperture 44 in composite thermoplastic elastomeric sheet 42 forming the blank is covered by web 49 having unitary protuberance 48 extending into aperture 44 and contacting beveled surface 46 which provides an annular contact surface or valve seat for protuberance 48. That is, web 49 is thicker in the region of aperture 44 and projects into the aperture. When a custom, valved cervical cap is fabricated using a cap blank of the type shown in FIGURE 16B, protuberance 48 will abut beveled surface 46 and thus not only will provide a larger sealing area but also, in the case of a relatively thin web, will prevent excessive deformation of the web itself during fabrication. Composite thermoplastic elastomeric sheet 42 is a laminate of two compatible yet dissimilar material layers 63 and 64 selected to provide the desired rigidity and hardness for the shell and the desired flexibility for the skirt portion of the cap.

The embossed region or land 58 in the blank derived from composite sheet 42 is provided by a substantially circular thermoplastic elastomer layer 63 superposed over and bonded to underlying layer 64.

Following vacuum molding in accordance with the procedures discussed with respect to Figures 5-8, the skirt portion is trimmed to the desired width. The skirt width, i.e., the distance between the junction of the shell and the skirt on one hand and the peripheral edge of the skirt on the other, depends on the depth of the fornices in any given instance, but usually is about 3 to about 7 millimeters.

Slits are cut intothe skirt portion of the formed cervical cap during the final trimming operation. Preferably the slits are substantially uniformly spaced from one another and extend about l.5 to about 5 millimeters from periphery 34, i.e., from the distal edge of the skirt portion.

Alternatively, a valved cervical cap can be first formed using a blank of substantially uniform thickness substantially as disclosed above and then a flexible skirt portion can be afixed thereto in the form of an elastomeric band that is bonded to the shell portion of the cap at the shell periphery by heat welding, ultrasonic techniques, adhesive bonding techniques, or the like. Spaced slits are provided in the band at the distal, i.e., unattached, edge thereof before or after attachment to the shell portion, as desired. The added skirt portion can be about 3 millimeters wide, or wider, depending on the depth of the fornices. Usually a skirt portion width of about 3 to about 7 millimeters is adequate. A relatively wide skirt portion may be added during fabrication and subsequently trimmed to a desired width for optimum fit. As pointed out hereinabove, the slits in the skirt portion can be spaced about 2 millimeters to about 10 millimeters apart, preferably about 4 to about 6 millimeters apart, and are at least about 1.5 millimeters long.

In addition to the blanks illustrated in FIGURES 15, 16A and 16B, a cervical cap embodying the present invention can also be fabricated using a blank of the type shown in FIGURES 9, 10 and 11. Referring to these latter FIGURES, circular blank 90 comprises an elastomeric web 91 that overlies and is secured to underlying planar thermoplastic, elastomeric sheet 93. If desired, elastomeric web 91 may also have a rectangular configuration, a polygonal configuration, or the like, as long as the margins of web 91 extend beyond sheet 93. As best seen in FIGURES 10 and 11, planar sheet 93 is circular and is provided with central aperture 95 having rounded or beveled edges. The outer peripheral margin 94 of planar sheet 93 is also beveled radially outwardly and away from web 91 so as to provide a relatively smooth transition between the downwardly depending skirt portion l02 of elastomeric web 91 and planar sheet 93 when blank 90 is transformed to a cap shape as described hereinabove.

Elastomeric web 91 is intermittently bonded or otherwise secured to planar sheet 93 so as to define therebetween a channel or a plurality of channels communicating with aperture 95 and periphery l03 of planar sheet 93. In other words, elastomeric web 91 is not continuously bonded to planar sheet 93 at periphery 103; however, there may be a plurality of spaced spot or linear bonding sites, if desired. In any case, the bonding sites are selected so as to provide a one-way passageway for fluids entering aperture 95 and exiting at periphery 103 as well as to provide a one-way valving action as a result of coaction between elastomeric web 91 and the portion of sheet 93 defining aperture 95.

One-way valving action is further enhanced by providing web 91 with integral protuberance 97 that extends at least partially into aperture 95 and sealingly abuts the aforementioned portion of sheet 93 that defines aperture 95 while permitting the opening of aperture 95 in response to a fluid pressure increase within the formed cap. Protuberance 97 can be made unitary with web 91.

To facilitate registry between web 91 and sheet 93 when composite blank 90 is assembled, one or more nipples such as nipple 98 may be provided on web 91 and corresponding complementary cavities such as cavity 96 in sheet 93.

The materials of construction for the elastomeric web and for the planar sheet, as well as their thickness ratios, can be the same as those discussed hereinabove in connection with the other embodiments of this invention. Preferably the thickness of the elastomeric web is about 0.1 to about 0.4 millimeters, more preferably about 0.2 to about 0.3 millimeters. The thickness of the planar sheet preferably is about 0.6 to about 2 millimeters, more preferably about 1 to about 1.5 millimeters. For blank 90, the diameter of circular, planar sheet 93 can be about 15 millimeters to about 50 millimeters, preferably about 25 millimeters to about 40 millimeters, depending on the desired size and depth of the cervical cap to be fabricated. The relative thicknesses of the web and the sheet also depend to some extent on the durometer hardness value of the particular material that is utilized. In general, the softer the material the greater can be the thickness of the web and/or the sheet.

A fabricated cervical cap embodying the present invention is shown in FIGURE 12. The shell portion of the cap 99 defines a concave inner surface of the cap and is entirely covered by elastomeric web portion l04 which defines the convex outer surface of cap 99. While the slits situated in skirt portion 102 in a spaced relationship to one another and extending into cap 99 from cap peripheral edge 100 can be substantially straight linear cuts, preferably these slits have an inverted-V configuration as illustrated by slits 101. In this particular embodiment skirt portion 102 is unitary with web portion 104.

The foregoing disclosure and the accompanying drawings are intended as illustrative and are not to be construed as limiting. Still other variations within the scope of the present invention as defined by the claims are possible and will readily present themselves to those skilled in the art.

## Claims

1. A valved cervical cap (10) comprising a cup-shaped elastomeric shell (22) having a convex outer surface (23) and a concave inner surface (24) which is substantially complementary with contiguous surface (26) of portio vaginalis cervicis in its actual configuration when in contact therewith, the shell (22) being provided with an aperture (27) at its apex, characterised in that the shell (22) is of sufficient depth to receive a major portion of the said portio but with the periphery (35) of the shell terminating short of fornices vaginae, and that an elastomeric web (29) over the aperture (27) and secured to the shell's outer surface (23) about the aperture defines a passageway communicating with the aperture and providing a discharge port (43) along the convex outer surface (23) and offset frm the aperture, the web (29) conforming to and coacting with a contiguous portion of the convex outer surface (23) to provide a one-way valve for uterine discharge entering the aperture (27) from within the shell (22).

2. A valved cervical cap according to claim 1, characterised by a skirt portion (30) integral with the shell and depending therefrom at the periphery of the shell, the skirt portion having a thickness which is less than the thickness of the shell and having a plurality of spaced-apart slits extending (32) from the peripheral edge (34) of the skirt portion (30).

3. A method of making a valved cervical cap (10) as claimed in claim 1 or claim 2, characterised by the steps of;
(a) applying a physiologically-tolerable, hardenable paste to cervix uteri in normal configuration and forming an impression of the cervix;
(b) replicating the cervix as a rigid mould;
(c) positioning a blank for a valved cervical cap made of a thermoplastic elastomeric material over the rigid mould;
(d) applying heat to the blank in an amount sufficient to soften the material;
(e) pressing the softened material against the rigid mould;
(f) cooling the pressed, softened material; and
(g) providing the resultant cap-shaped product with an aperture (27) at its apex and with an elastomeric web (29) over the aperture.

4. A method according to claim 3, characterised in that, after the formed article has been cooled and removed from the rigid mould, excess material is trimmed from the formed article to produce the valved cervical cap (10), and the shell periphery therof has a flexible skirt portion (30) attached thereto so as to depend from the shell.

5. A method according to claim 3 or claim 4, characterised by the use of an impression tray (65) suitable for making an impression of a cervix uteri which comprises:

(a) a hollow cup (67) for receiving the cervix and having a flexible wall terminating in a peripheral outwardly flared anterior lip and a bottom aperture;
(b) a posterior sleeve (70) integral with the cup (67) and surrounding the bottom aperture; and
(c) a hollow elongated stem (72) having a proximal end thereof received within the posterior sleeve (70) and defining a passageway communicating with the interior of the cup (67).

6. A method according to claim 5, characterised by the use of an implement for making an impression of a cervix uteri which comprises, in combination, the impression tray (65) and a syringe (59) having an elongated nozzle received within the tray, the syringe comprising a hollow body portion (52), a movable plunger (55) within the body portion, and an elongated nozzle (56) communicating with the body portion and adapted to be received within the passageway of the hollow elongated stem (72) of the impression tray (65).

## Patentansprüche

1. Mit Ventil versehene Cervix-Kappe (10) oder Pessar mit einer tassenförmigen elastomeren Schale (22), welche eine konvexe Außenfläche (24) aufweist, welche im wesentlichen komplementär zur anliegenden Oberfläche (26) der Portio Vaginalis Cervicis in ihrer tatsächlichen Form bei Berührung ausgebildet ist, wobei die Schale (22) mit einer Öffnung (27) an ihrem Grund versehen ist, dadurch gekennzeichnet, daß die Schale (22) eine ausreichende Tiefe aufweist, um den größeren Teil der Portio aufzunehmen, wobei jedoch der Rand (35) der Schale in kurzem Abstand von den Fornices Vaginae endet, und daß eine elastomere Membran (29) über der Öffnung (27) angeordnet und an der Außenfläche (23) der Schale die Öffnung umgebend befestigt ist und einen Durchgang bildet, welcher mit der Öffnung in Verbindung steht und eine Auslaßöffnung (43) längs der konvexen Außenfläche (23) gegenüber der Öffnung versetzt bildet, wobei die Membran (29) dem anliegenden Teil der konvexen Außenfläche (23) formmäßig entspricht und mit diesem in Berührung steht, um dadurch ein Einwegeventil für uterinären Ausfluß bildet, welcher vom Inneren der Schale (22) in die Öffnung (27) eintritt.

2. Mit Ventil versehene Cervix-Kappe nach Anspruch 1, dadurch gekennzeichnet, daß ein Schürzenabschnitt (30) einstückig mit der Schale ausgebildet ist und vom Rand der Schale nach unten absteht, wobei der Schürzenabschnitt eine Dicke aufweist, die geringer ist als die Dicke der Schale und mit einer Vielzahl von im Abstand angeordneten Schlitzen (32) versehen ist, welche von der Umfangskante (34) des Schürzenabschnitts (30) ausgehen.

3. Verfahren zur Herstellung einer mit Ventil versehenen Cervix-Kappe (10) nach Anspruch 1 oder 2, gekennzeichnet durch folgende Verfahrensschritte:
(a) Aufbringen einer physiologisch verträglichen aushärtbaren Paste auf den Cervix-Uteri in normaler Form und Bilden eines Abdrucks des Cervix.

(b) Herstellen eines Replikats des Cervix als starre Form;

(c) Anordnen eines Rohlings für eine mit Ventil versehene Cervix-Kappe aus einem thermoplastischen, elastomeren Material über der starren Form;

(d) Aufbringen von Wärme auf den Rohling in einer Menge, welche ausreicht, das Material zu erweichen;

(e) Andrücken des erweichten Materials gegen die starre Form;

(f) Abkühlen des verpreßten erweichten Materials und

(g) Versehen des erhaltenen kappenförmigen Erzeugnisses mit einer Öffnung (27) auf ihrem Grund und mit einer elastomeren Membran (29) über der Öffnung.

4. Verfahren nach Anspruch 3, dadurch gekennzeichnet, daß nachdem der geformte Gegenstand abgekühlt und von der starren Form abgenommen wurde, überschüssiges Material von dem geformten Gegenstand abgeschnitten wird, um die mit Ventil versehene Cervix-Kappe (10) zu erzeugen, wobei der Rand der Schale mit einem flexiblen Schürzenabschnitt (30) versehen wird, der nach unten von der Schale vorsteht.

5. Verfahren nach Anspruch 3 oder 4, dadurch gekennzeichnet, daß ein Abdruckgestell verwendet wird, welches geeignet ist, einen Abdruck des Cervix-Uteri zu machen, wobei das Gestell folgende Bestandteile aufweist:

(a) eine hohle Schale (87) zur Aufnahme des Cervix, welche eine flexible, am Umfang in einer nach außen abgeschrägten vorderen Lippe endende Wand und eine Bodenöffnung aufweist;

(b) eine hintere Hülse (70), welche einstückig mit der Schale (67) ausgebildet ist und die Bodenöffnung umgibt und

(c) einen hohlen, langgestreckten Stiel (72), dessen naheliegendes Ende in der hinteren Hülse (70) aufgenommen ist und einen Kanal definiert, welcher mit dem Inneren der Schale (67) in Verbindung steht.

6. Verfahren nach Anspruch 5, dadurch gekennzeichnet, daß ein Gerät zur Herstellung eines Abdrucks eines Cervix-Uteri verwendet wird, welches in der Kombination folgende Bestandteile aufweist: das Abdruckgestell (65) und eine Spritze (59) mit einer langgestreckten Düse, die in dem Gestell aufgenommen ist, wobei die Spritze einen hohlen Gehäuseabschnitt (52), einen beweglichen Kolben (55) in dem Gehäuseabschnitt und eine langgestreckte Düse (56) aufweist, die mit dem Gehäuseabschnitt in Verbindung steht und derart ausgebildet ist, daß sie in dem Kanal des hohlen langgestreckten Stiels (72) des Abdruckgestells (65) aufgenommen werden kann.

**Revendications**

1. Diaphragme (10) à clapet comprenant une coque élastomérique (22) en forme de coupelle ayant une surface extérieure convexe (23) et une surface intérieure concave (24) qui est sensiblement complémentaire d'une surface contiguë (26) de la portion intravaginale du col utérin dans sa configuration réelle lorsqu'elle est en contact avec elle, la coque (22) présentant une ouverture (27) à son sommet, caractérisé en ce que la coque (22) est d'une profondeur suffisante pour recevoir une partie majeure de ladite portion, mais la périphérie (35) de la coque se terminant à peu de distance du cul-de-sac du vagin, et en ce qu'un voile élastomérique (29), situé au dessus de l'ouverture (27) et fixé à la surface extérieure (23) de la coque, autour de l'ouverture, définit un passage communiquant avec l'ouverture et formant un orifice (43) d'écoulement le long de la surface extérieure convexe (23) et décalé par rapport à l'ouverture, le voile (29) se conformant à et coopérant avec une partie contiguë de la surface extérieure convexe (23) pour constituer un clapet unidirectionnel pour les écoulements utérins entrant dans l'ouverture (27) à partir de l'intérieur de la coque (22).

2. Diaphragme à clapet selon la revendication 1, caractérisé par une partie de jupe (30) réalisée d'une seule pièce avec la coque et partant vers le bas de celle-ci à la périphérie de la coque, la partie de jupe ayant une épaisseur qui est inférieure à l'épaisseur de la coque et présentant plusieurs fentes espacées (32) s'étendant à partir du bord périphérique (34) de la partie de jupe (30).

3. Procédé de fabrication d'un diaphragme à clapet (10) selon la revendication 1 ou la revendication 2, caractérisé par les étapes qui consistent:

(a) à appliquer une pâte durcissable physiologiquement tolérable sur le col de l'utérus en configuration normale et à former une empreinte du col;

(b) à former une réplique du col sous forme d'un moule rigide;

(c) à placer une ébauche d'un diaphragme à clapet réalisé en une matière élastomérique thermoplastique sur le moule rigide;

(d) à appliquer de la chaleur à l'ébauche en une quantité suffisante pour ramollir la matière;

(e) à presser la matière ramollie contre le moule rigide;

(f) à refroidir la matière ramollie et pressée; et

(g) à munir le produit résultant, en forme de coupelle, d'une ouverture (27) à son sommet et d'un voile élastomérique (29) au-dessus de l'ouverture.

4. Procédé selon la revendication 3, caractérisé en ce que, après que l'article formé a été refroidi et retiré du moule rigide, la matière excédentaire est arasée de l'article formé pour produire le diaphragme à clapet (10), et la périphérie de sa coque comporte une partie de jupe flexible (30) qui lui est attachée de façon à s'étendre vers le bas de la coque.

5. procédé selon la revendication 3 ou la revendication 4, caractérisé par l'utilisation d'un porte-empreinte (65) convenant à la réalisation d'une empreinte d'un col d'utérus, qui comprend

(a) une coupelle creuse (67) destinée à recevoir le col et ayant une paroi flexible se terminant par une lèvre antérieure périphérique évasée vers l'extérieur et une ouverture inférieure;

(b) un manchon postérieur (70) réalisé d'une seule pièce avec la coupelle (67) et entourant l'ouverture inférieure; et

(c) une tige creuse allongée (72) ayant une extrémité proximale reçue dans le manchon postérieur (70) et définissant un passage communiquant avec l'intérieur de la coupelle (67).

6. Procédé selon la revendication 5, caractérisé par l'utilisation d'un instrument pour réaliser une empreinte d'un col d'utérus, qui comprend, en combinaison, le porte-empreinte (65) et une seringue (59) comportant une buse allongée reçue à l'intérieur du porte-empreinte, la seringue comprenant une partie de corps creuse (52), un plongueur (55) mobile à l'intérieur de la partie de corps, et une buse allongée (56) communiquant avec la partie de corps et conçue pour être reçue dans le passage de la tige creuse allongée (72) du porte-empreinte (65).

Fig.1.

Fig.2.

Fig.3.

Fig.4A.

Fig.4B.

Fig.5.

Fig.6.

Fig.7.

Fig.8.

Fig.9.

Fig.10.

Fig.11.

Fig.12.

Fig. 13.

16

15

14

15

24

18

10

21

30

28

11

19

23

25

12

10

22

26

26

29  47  27  26

Fig. 14.

22  33  31  10

23

25

30

29

28

35

32  43  34  32

40

Fig. 15.

31

37

27

41

33

29

16.

16.

51

39

43

Fig. 16A.

41  29

51  40

27  45  39

Fig. 16B.

49  58  63

48  46  64